# EUROPEAN PATENT SPECIFICATION

(11) **EP 1 570 052 B1**
(45) Date of publication and mention of the grant of the patent: **21.01.2009**
(21) Application number: 03812367.5
(22) Date of filing: 02.12.2003
(51) Int. Cl.: C12N 5/08, A61K 35/30, A61P 25/00

(54) **METHOD FOR CULTURING NEURAL STEM CELLS USING HEPATOCYTE GROWTH FACTOR**
VERFAHREN ZUR KULTIVIERUNG NEURALER STAMMZELLEN UNTER VERWENDUNG DES HEPATOZYTENWACHSTUMSFAKTORS
PROCEDE DE CULTURE DE CELLULES SOUCHES NEURALES AU MOYEN DU FACTEUR DE CROISSANCE HEPATOCYTE

(30) Priority: 02.12.2002 US 430431 P
(43) Date of publication of application: 07.09.2005
(73) Proprietor: AnGes MG, Inc., Ibaraki-shi Osaka 567-0085 (JP)
(72) Inventor: KOKUZAWA, Jouji, Gifu-shi, Gifu 500-8287 (JP); YOSHIMURA, Shinichi, Motosu-gun, Gifu 501-0425 (JP); KITAJIMA, Hideomi, Gifu-shi, Gifu 500-8864 (JP); SHINODA, Jun, Kakamigahara-shi, Gifu 504-0006 (JP); KAKU, Yasuhiko, Gifu-shi, Gifu 502-0071 (JP); IWAMA, Toru, Gifu-shi, Gifu 502-0814 (JP); MORISHITA, Ryuichi, Osaka-shi, Osaka 532-0003 (JP); KUNISADA, Takahiro, Gifu-shi, Gifu 502-0071 (JP); SAKAI, Noboru, Gifu-shi, Gifu 500-8207 (JP)
(74) Representative: Grünecker, Kinkeldey, Stockmair & Schwanhäusser Anwaltssozietät
(86) International application number: PCT/JP2003/015401
(87) International publication number: WO 2004/050865

(56) References cited:
- WO-A-00/47238
- WO-A-99/11758
- US-A1- 2002 164 309
- KARP SHARON L ET AL: "Epithelial differentiation of metanephric mesenchymal cells after stimulation with hepatocyte growth factor or embryonic spinal cord" PROCEEDINGS OF THE NATIONAL ACADEMY OF SCIENCES OF THE UNITED STATES OF AMERICA, vol. 91, no. 12, 1994, pages 5286-5290, XP002274413 & ISSN: 0027-8424
- KOKUZAWA JOUJI ET AL: "Hepatocyte growth factor promotes proliferation and neuronal differentiation of neural stem cells from mouse embryos." MOLECULAR AND CELLULAR NEUROSCIENCE, vol. 24, no. 1, September 2003 (2003-09), pages 190-197, XP002274414 & ISSN: 1044-7431

## Description

### Technical Field

The present invention relates to the use of hepatocyte growth factor (HGF) for culturing neural stem cells (NSCs). The present invention further relates to a method for culturing cells using the culture medium comprising HGF.

### Background Art

To date, disorders of the central nervous system (CNS) are primarily treated through the administration of pharmaceutical compounds. Unfortunately, this kind of treatment has problems, such as limited ability for transporting pharmaceutical compounds across the blood-brain barrier and drug-resistance acquired by long-term administration of the compounds.

Thus, neurological tissue grafting is a promising technique for treating CNS disorders. Neurotransplantation avoids the need for constant drug administration as well as complicated drug delivery systems. However, the disadvantage is that neurotransplantation requires the use of cells that do not give rise to an immune reaction in the host and that are able to form normal neuronal connections with surrounding cells. To date, such cells are restricted to fetal cells in the initial studies (e.g., Perlow et al., Science 204: 643-647 (1979); Freed et al., N. Engl. J. Med. 327: 1549-1555 (1992); Spencer et al., N. Engl. J. Med. 327: 1541-1548 (1992); Widner et al., N. Engl. J. Med. 327: 1556-1563 (1992)). The use of fetal tissues is associated with ethical and political problems. Furthermore, more than one cell type constitutes the fetal CNS tissue and the tissues may be already infected with bacteria or virus. Therefore, transplantation of such tissue can involve some risk. Moreover, tissues from 6 to 8 fetuses are required to treat a single patient with N-methyl-4-phenyl-1,2,3,6-tetrahydropyridine (NPTP) -induced Parkinsonism (Widner et al., N. Engl. J. Med. 327: 1556-1563 (1992)). Thus, it is difficult to provide the constant supply of fetal tissue required for transplantation.

To overcome these drawbacks, researchers have suggested that multipotent neural stem cells (NSCs) may be induced to proliferate and provide a reliable source of an unlimited number of neural cells for neurotransplantation that are capable of differentiating into neurons, astrocytes and oligodendrocytes through use of a composition comprising a culture medium supplemented with epidermal growth factor (EGF) or transforming growth factor alpha (TGFα) (US Patent No. 5,851,832) or EGF, FGF-2 and LIF (WO 99/11758).

Neural stem cells (NSCs) possess the ability to self-renew and give rise to various types of neurons, astrocytes and oligodendrocytes in vitro and thus may play a major role both in the development and function of the mammalian central nervous system (CNS) throughout adulthood (Reynolds and Weiss, Science 255: 1707-1710 (1992); Temple and Devis, Development 120: 999-1008 (1994); Reynolds and Weiss, Dev. Biol. 175: 1-13 (1996); Palmer et al., Mol. Cell. Neurosci. 8: 389-404 (1997)) . The control of proliferation and differentiation of NSCs is an important hurdle in the development of transplantation strategies and other therapeutic approaches in treating neuronal injuries and neurodegenerative diseases (Svendsen et al., Prog. Brain Res. 128: 13-24 (1997); Armstrong et al., Cell Transplant 9: 55-64 (2000)).

Upon cultivation of NSCs in suspension in a media supplemented with nerve growth factor (s) lacking serum, the cells are known to form spherical cell clusters called neurospheres. The cells of the formed neurospheres are undifferentiated and one neurosphere is a progeny of one NSC. The cells of the neurospheres persistently proliferate in media containing one or more nerve growth factors (e.g., EGF, bFGF or combination thereof). Under differentiating conditions, the cells have the ability to differentiate into neural cells, such as neurons and glial cells (astrocytes and oligodendrocytes).

*In vitro* studies of NSC-based neurogenesis and gliogenesis suggest that these processes occur by step-wise restriction and depend on environmental signals (Ahmed et al., J. Neurosci. 15: 5765-5778 (1995); Tropepe et al., J. Neurosci. 17: 7850-7859 (1997); Arsenijevic and Weiss, J. Neurosci. 18: 2118-2128 (1998); Qian et al., Neuron 28: 69-80 (2000)). A number of growth factors support the proliferation of NSCs and the differentiation from their progenitors. For example, epidermal growth factor (EGF) and fibroblast growth factor-2 (FGF-2) are known to play important roles in the proliferation and maintenance of NSCs.

Recently, other factors, such as ciliary neurotrophic factor (CNTF) and insulin-like growth factor-1 (IGF-1), have been reported to function as key players in the control of NSC proliferation and maintenance (Arsenijevic et al., J. Neurosci. 21: 7194-7202 (2001); Shimazaki et al., J. Neurosci. 21: 7642-7653 (2001)). FGF-2 and platelet derived growth factor (PDGF) are also known to enhance neuronal differentiation (Johe et al., Genes Dev. 10: 3129-3140 (1996); Erlandsson et al., J. Neurosci. 21: 3483-3491 (2001); Yoshimura et al., Proc. Natl. Acad. Sci. USA 98: 5874-5876 (2001)). On the other hand, CNTF and bone morphogenetic protein (BMP) have been shown to enhance astrocyte differentiation in culture (Johe et al., Genes Dev. 10: 3129-3140 (1996); Bonni et al., Science 278: 477-483 (1997); Shimazaki et al., J. Neurosci. 21: 7642-7653 (2001)), whereas triiodothyronine (T3) has been shown to promote oligodendrocyte differentiation (Johe et al., Genes Dev. 10: 3129-3140 (1996)).

FGF-2, CNTF, leukemia inhibitory factor (LIF), brain-derived neurotrophic factor (BDNF) and PDGF have been classified as neurotrophic factors that play essential roles in the development, maintenance, activity-dependent modulation and regulation of the nervous system.

Hepatocyte growth factor (HGF) was first identified as a potent mitogen for hepatocytes and later purified and molecularly cloned in 1989 (Nakamura et al., Nature 342: 440-443 (1989)). HGF has various effects not only on hepatocytes but also on various types of cells.

Recent extensive analyses of HGF have revealed that HGF is a pleiotrophic factor that induces a variety of responses in normal development and pathological situations (Matsumoto and Nakamura, Biochem. Biophys. Res. Commun. 239: 639-644 4 (1997)) . HGF is also suggested to play a role during the early stages of neuronal induction. HGF is a polypeptide growth factor that acts by binding to the c-Met tyrosine kinase receptor. HGF and c-Met have been found to exist in developing and mature CNS (Jung et al., J. Cell. Biol. 126: 485-494 (1994); Honda et al., Mol. Brain Res. 32: 197-210 (1995); Hamanoue et al., J. Neurosci. Res. 43: 554-564 (1996); Achim et al., Dev. Brain Res. 102: 299-303 (1997)). The expression of both HGF and c-Met persists in adult (Jung et al., J. Cell. Biol. 126: 485-494 (1994); Achim et al., Dev. Brain Res. 102: 299-303 (1997); Streit et al., Development 124: 1191-1202 (1997); Maina and Klein, Nat. Neurosci. 2: 213-217 (1999)). Thus, HGF is a pleiotrophic cytokine that induces mitogenesis, motility, morphogenesis and antiapoptotic activities of neural cells (Honda et al., Mol. Brain Res. 32: 197-210 (1995); Hamanoue et al., J. Neurosci. 43: 554-564 (1996); Ebens et al., Neuron 17: 1157-1172 (1996); Novak et al., J. Neurosci. 20: 326-337 (2000)). Furthermore, HGF has recently been shown to be expressed in different parts of the nervous system and to have neurotrophic ability. However, the effect of HGF on the proliferation or differentiation of NSCs is unknown.

### Disclosure of the Invention

The present inventors examined the in vitro effect of HGF on the proliferation and differentiation of NSCs isolated from E14 mouse striatal cells. Medium containing HGF alone was capable of inducing neurosphere formation from the striatal cells. The addition of HGF to culture medium containing either FGF-2, EGF, or both was shown to increase both the size and number of newly formed neurospheres. More neurons can be obtained by adding HGF to a differentiation medium containing 1% fetal bovine serum. In contrast, the number of neurospheres was shown to be reduced after repeated subculture with mechanical dissociation of NSCs. This suggests that HGF-formed neurospheres are predominantly composed of progenitor cells committed to neuronal or glial lines. These results in turn suggest that HGF promotes proliferation and neuronal differentiation of NSCs derived from mouse embryos.

Thus the present description provides the following:
(1) a growth medium for culturing neural stem cells (NSCs) which comprises hepatocyte growth factor (HGF);
(2) the growth medium of (1), which further comprises another growth factor in addition to HGF;
(3) the growth medium of (2), wherein the growth factor is selected from the group of fibroblast growth factor-2 (FGF-2) and/or epidermal growth factor (EGF);
(4) the growth medium of (1) or (2), wherein the medium is used for *in vitro* proliferation of mammalian NSCs;
(5) the growth medium of (1) or (2), wherein the medium is used for *in vitro* differentiation of mammalian NSC;
(6) a method for culturing NSCs wherein an NSC or a population of cells comprising at least one NSC is cultured in the growth medium of any one of (1) to (5);
(7) a method for proliferating NSCs wherein an NSC or a population of cells comprising at least one NSC is cultured in the growth medium of any one of (1) to (5) under conditions that allow the proliferation of the NSC;
(8) a method for differentiating NSCs wherein an NSC or a population of cells comprising at least one NSC is cultured in the growth medium of any one of (1) to (5) under conditions that allow the differentiation of the NSC into a population of cells containing neurons and glia cells;
(9) the method of any one of (6) to (8), wherein the NSC is derived from mammalian neural tissue selected from the group consisting of brainstem, cerebellum, cerebral cortex, midbrain, spinal cord and ventricular;
(10) the method of any one of (6) to (9), wherein the NSC is genetically modified;
(11) use of a cell population obtained by culturing an NSC or a population of cells comprising at least one NSC in the medium of any one of (1) to (6) for treating a neurological disorder;
(12) the use of (11), wherein the cell population is enriched in NSCs;
(13) the use of (11), wherein the cell population is enriched in neurons; and
(14) the use of any one of (11) to (13), wherein the neurological disorder is selected from the group of epilepsy, head trauma, stroke, amyotrophic lateral sclerosis, Parkinson's disease, Alzheimer's disease and Huntington's disease.
(15) the use of any one of (11) to (14), wherein the neurological disorder is treated by transplantation of at least one cell of the cell population.

The present invention provides a method for culturing neural stem cells comprising the step of culturing a neural stem cell or a population of cells comprising at least one neural stem cell in a growth medium comprising hepatocyte growth factor (HGF), with the proviso that the neural stem cell or a population of cells comprising at least one neural stem cell is not obtained from human embryo.

The present invention also provides a method for proliferating neural stem cells comprising the step of culturing a neural stem cell or a population of cells comprising at least one neural stem cell in a growth medium comprising HGF under conditions that allow the proliferation of the neural stem cell, with the proviso that the neural stem cell or a population of cells comprising at least one neural stem cell is not obtained from human embryo.

The present invention further provides a method for differentiating neural stem cells comprising the step of culturing a neural stem cell or a population of cells comprising at least one neural stem cell in a growth medium comprising HGF under conditions that allow the differentiation of the neural stem cell into a population of cells containing neurons and glia cells, with the proviso that the neural stem cell or a population of cells comprising at least one neural stem cell is not obtained from human embryo.

In certain embodiments of the methods provided by the present invention, the growth medium further comprises (i) fibroblast growth factor-2 (FGF-2), (ii) epidermal growth factor (EGF), or (iii) FGF-2 and EGF, in addition to HGF.

In certain embodiments of the methods provided by the present invention, the neural stem cell is derived from mammalian neural tissue selected from the group consisting of brainstem, cerebellum, cerebral cortex, midbrain, spinal cord and ventricular.

In certain other embodiments of the methods provided by the present invention, the neural stem cell is genetically modified.

### Brief Description of the Drawing

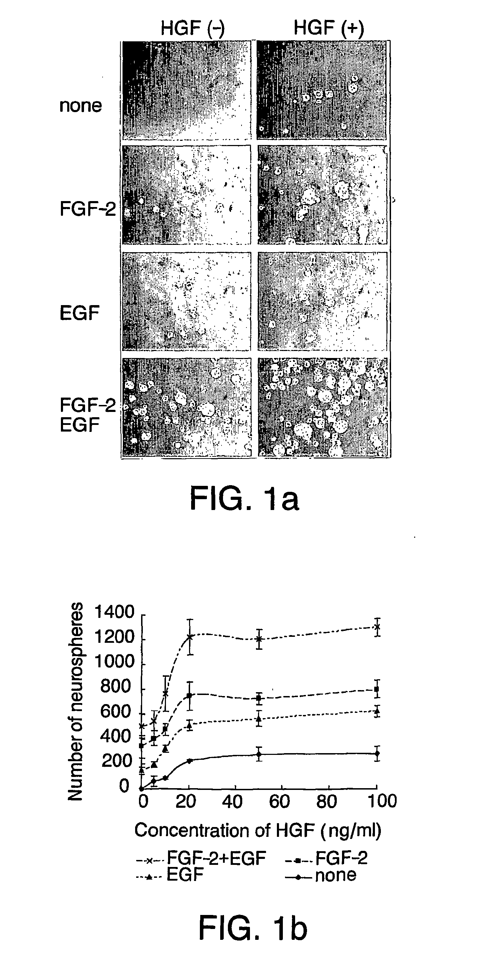

Figs. 1a - 1b show the effect of HGF on the formation of neurospheres isolated from mouse E14 striatal cells. Fig. 1a depicts photographs of primacy spheres grown in the presence of various growth factors for 7 days. Scale bar: 50 µm. Fig. 1b depicts a graph showing the number of neurospheres in E14 striatal cells. In the presence of FGF-2 (■), EGF (▲), FGF-2 plus EGF (x) or none (◆), and various concentrations of HGF, 75,000 cells per well were incubated in a 24-well plate for 7 days. Mean values of five different experiments are indicated.

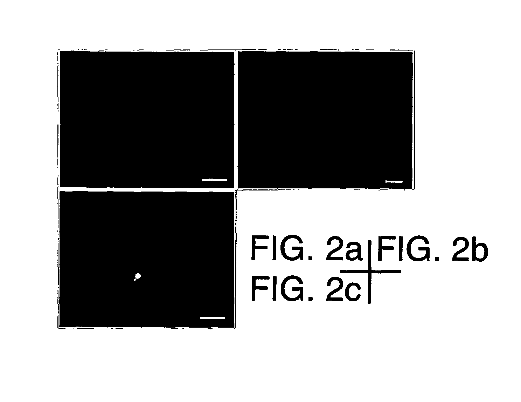

Figs. 2a - 2c depict photographs showing the result of immunostaining of c-Met receptor (a, b) and nestin c. Fig. 2a shows the c-Met receptor expression (green) on the cells in the neurosphere cultured in the presence of FGF-2 and EGF. The nuclei of the cells were counterstained with Hoechst (blue). Scale bar: 20 µm. Fig. 2b shows the immunostaining of c-Met receptor in single dissociated cells. Scale bar: 10 µm. Fig. 2c shows the expression of nestin (red) in the cells in the neurosphere under the presence of HGF. Scale bar: 50 µm.

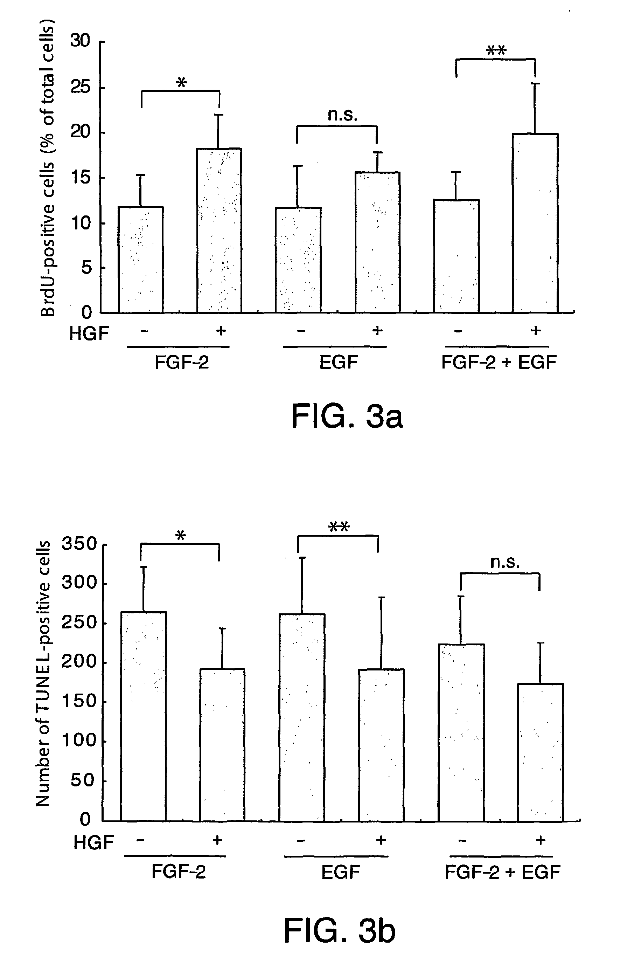

Fig. 3a shows the percentage of BrdU positive cells in the neurospheres cultured in the presence of various growth factors. BrdU (10µM) was added to the secondary neurospheres, and the neurospheres were incubated for 12 hr. Then the neurospheres were mechanically dissociated, plated on 24-well plates and fixed 12 hr later. Mean values of five different experiments are indicated. *: p<0.05 versus FGF, ** : p<0.01 FGF + EGF. Fig. 3b shows the number of TUNEL positive cells in the neurospheres with or without HGF. Secondary neurospheres were mechanically dissociated, fixed in 1% paraformaldehyde and stained using the TUNEL kit. Mean values of five different experiments are indicated. *: p<0.05 versus FGF, **: p<0.05 versus EGF.

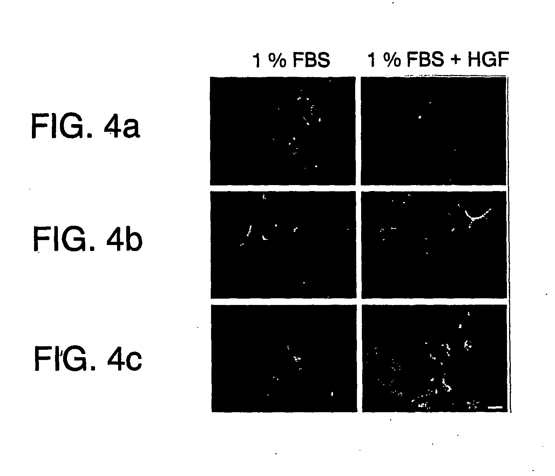

Fig. 4 depicts photographs showing the effect of HGF on the percentage of phenotypes. Double-labeled immunocytochemistry of cells from neurospheres incubated in the presence of HGF, FGF-2 plus EGF, and FGF-2 plus EGF plus HGF are shown. The cells were plated in 1% FBS or 1% FBS plus HGF. MAP-2 positive neurons (red), GFAP positive astrocytes (green) and Hoechst labeling nuclei (blue) are shown. Scale bar, 50 µm.

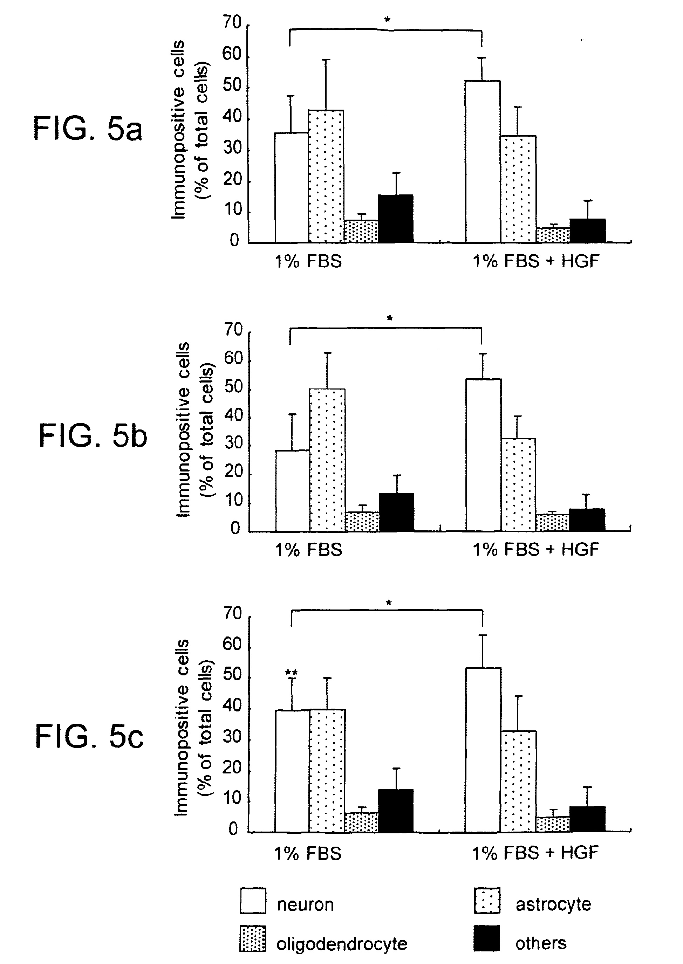

Fig. 5 depicts graphs showing the percentage of immunopositive cells among the neurospheres cultured in the presence of HGF (a), FGF-2 and EGF (b), FGF-2, EGF and HGF (c). The neurospheres were differentiated in 1% PBS or 1% PBS plus HGF (20 ng/ml) for 7 days. Mean values of five different experiments are indicated. *: p<0.01 versus 1% FBS neuron, **: p<0.01 versus (b).

### Best Mode for Carrying out the Invention

The words "a", "an" and "the" as used herein mean "at least one" unless otherwise specifically indicated.

The establishment of *in vitro* culture systems for the expression of neural stem cells (NSCs) using FGF-2 and/or EGF as a mitogen provides a useful model for examining cellular mechanisms underlying the development of the NSC (Reynolds and Weiss, Science 255: 1707-1710 (1992); Temple and Davis, Development 120: 999-1008 (1994); Reynolds and Weiss, Dev. Biol. 175: 1-13 (1996); Weiss et al., J. Neurosci. 16: 7599-7609 (1996); McKay, Science 276: 66-71 (1997); Palmer et al., Mol. Cell. Neurosci. 8: 389-404 (1997)). It is important to elucidate the mechanisms of NSC proliferation and differentiation for novel future therapeutic approaches against neuronal injuries and neurodegenerative diseases. Recent studies have shown that environmental signals, such as cytokines and growth factors, influence the proliferation or differentiation of NSCs (Ahmed et al., J. Neurosci. 15: 5765-5778 (1995); Tropepe et al., J. Neurosci. 17: 7850-7859 (1997); Tropepe et al., Dev. Biol. 208: 166-188 (1999); Arsenijevic and Weiss, J. Neurosci. 18: 2118-2128 (1998); Qian et al., Neuron 28: 69-80 (2000). The present study was conducted to determine the *in vitro* effect of hepatocyte growth factor (HGF) on NSCs.

In the present study, the immunoreactivity of c-Met receptor was observed on cells in a neurosphere isolated from E14 mouse embryos. This suggests that the receptors for HGF exist on the cells in neurospheres. Without FGF-2 or EGF, neurosphere formation was observed in medium containing HGF alone, although the inclusion of FGF-2, EGF, or both in the medium resulted in neurospheres larger in size and having a greater number of cells therein. Neurospheres formed with HGF contained cells that were immunopositive for nestin, and multipotent (i.e., capable of differentiating into neurons, astrocytes and oligodendrocytes). However, the ability to form neurospheres was reduced after repeated subculture following mechanical dissociation of the neurospheres. This phenomenon may be explained by presuming that HGF not only promotes the division of stem cells but also the production of progenitor cells that are finally differentiated into neurons and glia to various degrees. In fact, it has been reported that neurospheres contain stem cells as well as neuronal and glial progenitor cells already committed to neuron and glia (Reynolds and Weiss, Dev. Biol. 175: 1-13 (1996); Svendsen and Caldwell, Prog. Brain Res. 127: 13-24 (2000)).

The proliferative division of NSC is classified into symmetric division to produce NSCs and asymmetric division to produce progenitor cells. It is also known that neuronal progenitor cells are mainly produced in the early stage (neurogenic phase) and that glial progenitor cells are produced in the later phase (gliogenic phase) (Morrison et al., Cell 88: 287-298 (1997); Qian et al., Neuron 28: 69-80 (2000)). It is likely that neurospheres formed in the presence of HGF contain predominantly progenitor cells committed to neuron or glia. These progenitor cells cannot be discriminated from NSCs since they are also immunopositive for nestin. In other words, HGF promotes asymmetric division rather than symmetric division. This presumption explains the reduced ability the cells isolated in the medium containing HGF to self-renew.

According to the present study, the addition of HGF to a culture medium containing FGF-2, EGF, or a combination thereof, increased the number and size of neurospheres. Though not wishing to be bound by theory, this phenomenon may be explained by the hypotheses that (1) HGF promotes proliferation of NSCs; (2) HGF inhibits apoptosis or necrosis of NSCs; and/or (3) HGF maintains NSCs in an undifferentiated condition.

In support of hypothesis (1), the addition of HGF to the medium containing FGF-2, EGF, or both was shown to increase the number of BrdU-positive cells. Furthermore, more neurons were obtained after culture in the growth medium containing HGF. These results suggest that HGF may promote asymmetric division wherein neuronal progenitor cells are produced. In relation to hypothesis (2), it has been reported that a significant amount of cell death occurs during the development of CNS (Oppenheim, Annu. Rev. Neurosci. 14: 453-501 (1991)). Interestingly, most of the TUNEL-positive cells were located in the periventricular zone (PVZ) of the brain where NSCs exist (Thomaidou et al., J. Neurosci. 17: 1075-1085 (1997); Blaschke et al., Development 122: 1165-1174 (1996)). It is also known that cell death occurs at the center of neurospheres during both growth and differentiation conditions. Apoptosis appears to be closely involved in this cell death. According to the present study, it was shown that there are many TUNEL-positive cells in neurospheres cultured in growth medium. The anti-apoptotic effect of HGF may lead to an increase in both the number and size of neurospheres, in addition to the proliferative effects of NSCs as mentioned above. Regarding hypothesis (3), it has been reported that signal transduction occurs through gb130 activated by CNTF (Shimazaki et al., (2001)) or expression of Hes1 or Hes5 by Notch signaling (Artavanis-Tsakonas et al. , Science 284 : 770-776 (1999); Nakamura et al., J. Neurosci. 20: 283-293 (2000); Ohtsuka et al., J. Biol. Chem. 276: 30467-30474 (2001)).

According to the present invention, HGF was found to promote proliferation and neuronal differentiation of NSCs. Thus, the present description provides a growth medium supplemented with HGF for culturing NSCs. The growth medium may be used for in vitro proliferation or differentiation of mammalian NSCs.

HGF is a heterodimer with a molecular weight of 82, 000 to 85,000, consisting of alpha and beta chains. The nucleotide sequence and amino acid sequence of human HGF is known in the art (Nakamura et al., Nature 342: 440-443 (1989)). Any HGF, including analogues, homologues and mutants, may be used in the growth media of the present invention so long as it retains its ability to induce proliferation and/or differentiation of NSCs. Thus, apart from the above-mentioned human HGF, those HGF homologues derived from mammals other than human may be used in the present invention. In addition, since proteins encoded by genes with similar sequences are known to have similar activities, proteins encoded by genes or polynucleotides that hybridize under stringent conditions to the human HGF gene may also be used for the present invention, provided such proteins are capable of inducing proliferation and/or differentiation of NSCs. Furthermore, the HGF used in the present invention may be a mutant of HGF occurring in nature or one arising from modifications, such as by deletion(s), substitution(s), addition(s) and/or insertions(s) of one or more amino acid residues. Fragments of HGF may also be used in the present invention so long as they induce proliferation and/or differentiation of NSCs.

HGF, and analogues, homologues and mutants thereof may be isolated from natural sources according to conventional methods, for example, using an anti-HGF antibody or based on their activity. Alternatively, they may be expressed as recombinant proteins and then purified as needed. For recombinant expression, genes encoding HGF may be obtained based on known techniques, such as site-directed mutagenesis, polymerase chain reaction (PCR) (see, for example, edit. Ausubel et al., Current Protocols in Molecular Biology, publish. John Wiley & Sons, Section 6.1-6.4 (1987)) and hybridization (see, for example, edit. Ausubel et al., Current Protocols in Molecular Biology, publish. John Wiley & Sons, Section 6.3-6.4 (1987)).

HGF is added to the medium used in the present invention at a final concentration of about 1 ng/ml to about 1 mg/ml, preferably about 1 ng/ml to about 100 ng/ml, and more preferably about 1 ng/ml to about 20 ng/ml. However, the optimal concentration of HGF differs in relation to the addition of other growth factors. Generally, it is preferable to add growth factors at a total concentration of about 1 ng/ml to about 1 mg/ml, and usually concentrations of about 1 ng/ml to about 100 ng/ml are sufficient. To determine the optimal concentration of HGF and other particular growth factors, those skilled in the art can easily perform simple titration experiments. Such is a matter of routine experimentation.

A growth medium used in the present invention may comprise other factors required for culturing NSCs. Namely, any known culture medium may be used in the present invention, so long as it supports growth of NSCs. Examples of suitable culture media include, but are not limited to, DMEM, F-12, HEM, RPIM, etc. Two or more of these media may be used in combination as described in the Examples, infra. The combination of DMEM and F-12 comprising HGF used in the Examples is a particularly preferred example of a culture medium used in the present invention.

If required, supplements such as amino acids (e.g., glutamine, etc.), vitamins, minerals, proteins (e.g., transferring, etc.) and antibiotics (e.g., penicillin, streptomycin, gentamicin, etc.) may be added to the medium. A growth medium used for proliferation of NSC preferably is a serum-free culture medium, since serum tends to induce differentiation of NSCs. Alternatively, those used for differentiation of NSC may optionally contain serum. Exemplary sera include those derived from bovine, chicken, equine, etc. The serum may be added at a concentration ranging from about 0.01 to about 10 %, preferably about 0.1 to about 5 %, more preferably about 0.5 to about 3 %, and most preferably about 1.0 to about 1.5 %.

Furthermore, a growth medium used in the present invention may comprise other growth factor(s) in addition to HGF. Growth factors that may be used in combination with HGF include those that allow NSCs to proliferate. Examples include, but are not limited to, fibroblast growth factor-2 (FGF-2; also referred to as basic fibroblast growth factor (bFGF)), epidermal growth factor (EGF), amphiregulin, acidic fibroblast growth factor (aFGF or FGF-1), transforming growth factor α (TGFα), etc. In addition to the above-mentioned growth factors that induce proliferation of NSCs, other growth factors that influence proliferation and differentiation of NSC may also be added to a medium used in the present invention. For example, insulin-like growth factor (IGF-1), necrosis growth factor (NGF), platelet-derived growth factor (PDGF), thyrotropin releasing hormone (TRH), transforming growth factor β (TGFβ), etc., are known to influence proliferation and differentiation of cells, and may be added as needed. Furthermore, FGF-1, FGF-2, ciliary neurotrophic factor (CNTF), NGF, brain-derive neurotrophic factor (BDNF), neurotropin 2, neurotropin 4, interleukins, leukemia inhibitory factor (LIF), cyclic adenosine monophosphate, forskolin, tetanus toxin, high levels of potassium, amphiregulin, TGF-α, IGF-1, dexamethasone, isobutyl 3-methylxanthine, somatostatin, growth hormone, retinoic acid and PDGF are also known to influence the differentiation of NSCs, and thus may be added to a medium for differentiation.

These growth factors may be added alone or in combination with other growth factors to a medium of the present invention. As demonstrated in the Examples, preferred growth factors include FGF-2 and/or EGF. Similar to HGF, these additional growth factor(s) may comprise analogues, homologues, mutants and fragments thereof, so long as they possess the agility to enhance NSC proliferation and/or differentiation induced by the addition of HGF.

The present invention further provides a method for culturing NSCs. The addition of HGF to a growth medium was found to induce proliferation of a NSC. Thus, the present invention provides a method for proliferating NSCs. According to the method, an NSC or a population of cells comprising at least one NSC is cultured in a growth medium that comprises HGF. Furthermore, the present inventors discovered that, when HGF was added to the medium during differentiation of neurospheres, neurons were obtained more than astrocytes. Thus, the present invention provides a method for differentiating NSCs. According to the method, an NSC or a population of cells comprising at least one NSC is cultured in a growth medium that comprises HGF.

A neural stem cell (NSC) is an undifferentiated neural cell capable of self-maintenance. NSCs can be obtained from embryonic, post-natal, juvenile and adult neuronal tissues. The neuronal tissue can be obtained from any animal that has neuronal tissue. The neuronal tissue is preferably obtained from mammals, more preferably rodents and primates, and even more preferably, mice, rat and humans, provided it is not obtained from a human embryo. Suitable areas for obtaining the neuronal tissue include the brainstem, cerebellum, cerebral cortex, midbrain, spinal cord and ventricular tissue, and areas of peripheral nervous system, such as the carotid body and adrenal medulla. Preferred areas include, but are not limited to, those in the basal ganglia, for example, the striatum that consists of the caudate and putamen, and cells of the globus pallidus, nucleus basalis, substantia nigra pars compacta and subthalamic nucleus. Particularly preferred neural tissue is obtained from neural ventricular tissue, including the subependyma. Human NSCs may be derived from fetal tissue following elective abortion or from a post-natal, juvenile or adult organ donor. They may be obtained by biopsy, or from patients undergoing neurosurgery, such as epilepsy surgery, temporal lobectomies and hippocampalectomies.

Cells or cell populations that can be used for the methods of the present invention may be obtained from above-mentioned tissues through dissociation using any method known in the art. Methods for dissociating cells from connecting extracellular matrix include enzyme treatments, such as treatment with trypsin or collagenase, and physical methods, such as those using blunt instrument.

If required, the neural stem cell may be genetically modified. The phrase "genetic modification" refers to stable or transient alteration of the genotype of the cell by introduction of at least one exogeneous nucleic acid construct. A preferred nucleic acid construct includes a vector comprising a DNA encoding an object protein downstream of an expression regulatory sequence. Such vectors include viral vectors, plasmids, and the like. NSCs derived from transgenic animals are also included in the genetically modified NSCs.

Alteration of the genotype of an NSC can enable efficient detection of the cell. For example, when NSCs are introduced with a detectable reporter gene (e.g., β-galactosidase gene, green fluorescent protein gene, etc.) linked downstream of regulatory sequence that achieves specific expression in neurons, astrocytes or oligodendrocytes, the differentiation of the NSCs can be detected based on the reporter gene.

Alternatively, genes encoding a biologically active substance may be used for transfection to provide cells useful in the treatment of CNS disorders. Examples of biologically active substance include, but are not limited to, BDNF; CNTF; EGF; FGF-1; FGF-2; IGFs; interleukins; neurotrophins, such as NT-3, NT-4/NT-5 and such; NGF; PDGF; TGF-α; TGF-βs; receptors of them; receptors of neurotransmitters, such as acethylcholine (ACh), dopamine, endorphin, enkephalin, epinephtine, γ-aminobutyric acid (GABA), glutamate, glycine, histamine, L-3,4-dihydroxyphenylalanine (L-DOPA), N-methyl D-aspartate, norepinephrine, serotonin, substance-P and tachykinin; neurotransmitter synthesizing genes, such as choline O-acetyltransferase (ChAT), dopa decarboxylase (DDC), dopamine-β-hydroxylase (DBH), glutamic acid decarboxylase (GAD), histidine decarboxylase, phenylethanolamine N-methyltranferase (PNMT), tyrosine hydroxylase (TH) and tryptophan hydroxylase; and neuropeptide encoding genes, such as bombesin, calcitonin gene-related peptide, cholecystokinin (CCK), enkephalin, glucagons, neuropeptide-Y, somatostatin, substance-P, vasopressin and vasoactive intestinal peptide (VIP)

Neural cells can be cultured in suspension or on a fixed substrate. For proliferation of NSCs, culture in suspension is preferred due to the fact that substrates tend to induce differentiation of NSCs.

According to the present methods, a cell or cell suspension may be seeded in any container that can sustain the cell. Such containers include culture disks, culture flasks, culture plates, roller bottles, and the like. The container comprises a growth medium containing HGF of the present invention. Other growth factors and molecules that influence the proliferation and/or differentiation may be added alone or in various combination, either all together or in a temporal sequence.

Conditions close to physiological conditions should be used for the culture of the present invention. Thus, the optimum culture temperature ranges from about 30 °C to about 40 °C, more preferably from about 32 °C to about 38 °C, and more preferably from about 35°C to about 37 °C; likewise, the pH of the media is preferably between about pH 6 to about 8, and more preferably about pH 7.0 to about 7.8. However, culture conditions of the present invention are not restricted to these examples, and those skilled in the art can successfully determine adequate conditions considering the various parameters such as the kind of used media, the origin of cells, and the like.

The culture may be continued for sufficient time as required.

According to US Patent 5,851,832, proliferating neurospheres lift off the floor of the culture dish and tend to form free-floating clusters characteristic of neurospheres after about 4 to 5 days. Thus, for proliferation of NSCs, it may be preferred to continue the culture for a period longer than 4 days. The culture medium should be replaced every 2 to 7 days, preferably ever 2 to 4 days. Specifically, the culture is subjected to gentle centrifugation after about 3 to 10 days (more particularly after about 6 to 7 days) *in vitro,* and then resuspended in appropriate complete medium.

Differentiation of NSCs can be also conducted by any method known in the art. For example, US Patent 5,851,832 teaches liberation of inositol triphosphate and intracellular Ca²⁺ ; liberation of diacyl glycerol and the activation of protein kinase and other cellular kinases; treatment with phorbol esters, differentiation-inducing growth factors, collagen, fibronectin, laminin, MATRIGEL^{™} (Collaborative Research), and such; and plating the cells on a fixed substrate coated with an ionically charged surface, such as poly-L-lysine, poly-L-omithine, etc.

After 2 to 3 days of culture under differentiating conditions, NSCs tend to differentiate. The differentiation of NSCs may be assayed according to conventional methods. For example, nestin has been characterized as an intermediate filament protein found in many types of undifferentiated CNS cells (Lehndahl et al., Cell 60: 585-595 (1990)). Therefore, NSCs may be preferably detected immunocytochemically using an anti-nestin antibody. Alternatively, markers for neurons or glia cells are also known in the art. Markers for neurons include microtuble-associated protein 2 (MAP-2), neuron-specific enolase (NSE), neurofilament (NF), etc. Markers for glial cells include glial fibrillary acidic protein (GFAP)(an identifier of astrocytes), galactocerebroside (GalC)(a myelin glycolipid identifier of oligodendrocytes), myelin basic protein (MBP) (identifier of oligodendrocytes), O₄ (identifier of oligodendrocytes), etc. Various other markers for neurons and glia cells are known in the art, and any of them may be successfully employed for determining the differentiation of NSCs in the present invention. Many antibodies for detecting NSCs, neurons and glia cells, respectively, are commercially available and any of them may be used. Apart from immunocytochemistry, the markers may also be detected using cDNA and RNA probes specific for the markers of respective cells.

If required, the NSCs obtained by the culture or proliferation method of the present invention can be cryopreserved by any method known in the art.

Transplantation of tissue into the CNS is recognized as potential treatment for neurodegenerative disorders and CNS damage due to injury (Lindvall, TINS 14(8): 376-383 (1991)). Transplantation of new cells into damaged areas of the nervous system is considered to have the potential to restore damaged brain circuits and provide neurotransmitters, thereby restoring neurological function. Thus, a cell population obtained by the culture methods of the present invention may be used in the treatment of neurological disorders. The present description provides for the use of cell populations obtained by culturing an NSC or a population of cells comprising at least one NSC in a medium that comprises HGF to treat a neurological disorder. The cells obtained according to the present method are particularly preferred in that they have not been immortalized and are not of tumorigenic origin. Furthermore, in cases where the autologous tissue does not have a defect, cells to be cultured according to the present methods may be obtained from donor tissue to avoid occurrence of immune response.

Neurological disorders that can be treated by a cell or a cell population cultured according to the present invention include: neurodegenerative diseases, acute brain injuries and CNS dysfunctions. Degeneration of neural cells in a particular location of the CNS are observed in some disease, including Alzheimer's disease, amyotrophic lateral sclerosis, Huntington's disease, multiple sclerosis and Parkinson's disease. In Alzheimer's disease, cellular degeneration of the forebrain and cerebral cortex, and localized degeneration in the basal ganglia, particularly in the nucleus basalis of Meynert, are observed. Huntington's chorea is known to be associated with neuronal degeneration in the striatum; Parkinson's disease is associated with degeneration of dopamine neurons in an area of the basal dorsal stratum. Other forms of neurological impairment can occur as a result of neural degeneration, such as amyotrophic lateral sclerosis and cerebral palsy or as a result of CNS tauma, such as stroke and epilepsy.

In fact, transplantation of neurons has been suggested for patients with Huntigton's disease (Science 287: 5457 (2000)); likewise, neurons that produce dopamine are reported to be effective for Parkinoson's disease (Nature 418: 50-56 (2002)). Furthermore, transplantation of glia cells result in spinal cord repair (Honmou et al., J. Neurosci. 16(10): 3199-3208 (1996); Nishio et al., Physiological Sci. (2001)).

Contrary to the transplantation of mature neurons or glia cells, transplantation of undifferentiated NSCs results in in vivo differentiation that is expected to provide neurons functional in the CNS circuit or immature astrocytes. Immature astrocytes are known to possess a larger migration ability compared to mature astrocytes (Lindsay et al., Neurosci. 12: 513-530 (1984); Duffy et al., Exp. Cell. Res. 139: 145-157 (1982); WO 91/06631) and thus optimize the opportunity for oligodendrocyte growth and division at sites distant from the transplanted site.

The following examples are presented to illustrate the present invention and to assist one of ordinary skill in making and using the same. The examples are not intended to otherwise limit the scope of the invention in any way.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

### [Examples]

### Material and Method

### (1) Primary culture and neurosphere passage

Striatal cells were removed from 14 day old mouse embryos (C57 BL/6, plug day =1.0) in PBS buffer containing penicillin (50 U/ml) and streptomycin (50 U/ml) (both from ICN Pharmaceuticals). The tissue was mechanically dissociated with a fire-polished pipette in serum-free medium consisting of DMEM and F-12 nutrient (1:1; Invitrogen). The cells were grown in growth medium in Falcon culture flasks (Falcon), six-well dishes (Falcon) or 24-well dishes (Falcon) at a concentration of 150,000 cells/ml. The growth medium contained DMEM and F-12 nutrient (1:1; Invitrogen), glucose (0.6%), glutamine (2 mM), B27 supplement (2%; Invitrogen) and EGF, and FGF-2 and/or HGF (R&D Systems) at a concentration of 20 ng/ml each. Half of the medium was replaced every 4 days with fresh medium containing the same concentration of growth factors. After 7 days, primary neurospheres were collected by centrifugation (2,300 x g), resuspended in fresh medium, and dissociated with a fire-polished pipette as described above.

To assess the effect of HGF on the size and number of neurospheres in primary culture, E14 striatal cells were cultured for 7 days in growth medium containing FGF-2 (20 ng/ml) and/or EGF (20 ng/ml), supplemented with various concentrations of HGF. The number of primary neurospheres was counted, and the size of the primary neurospheres was measured under a phase-contrast microscope (IMT-2, Olympus, Japan).

### (2) Differentiation of neurospheres

The secondary neurospheres that were cultured in the presence of HGF alone, FGF-2 + EGF or FGF-2 + EGF + HGF were rinsed in growth medium lacking growth factors, and dissociated with a fire-polished pipette. Dissociated cells (1 x 10⁵ cells) were plated onto poly-D-lysine coated coverslip in 24-well plates (Falcon). To determine the effect of HGF on the differentiation of NSCs, each well contained 1% fetal bovine serum (FBS) and 20 ng/ml HGF or only 1% FBS. The cells were fixed with 4% paraformaldehyde in PBS containing 4% sucrose after 7 days.

### (3) Antibodies

Primary antibodies (final dilution; source): mouse monoclonal antibody against nestin (1:500; Chemicon), mouse monoclonal antibody against microtuble-associated protein 2 (MAP-2) (1:500; Sigma-Aldrich), rabbit polyclonal antibody against glial fibrillary acidic protein (GFAP) (1 : 500; DAKO), mouse IgM monoclonal antibody against O₄ (1:20; Chemicon), rabbit polyclonal antibody against c-Met (1:100; Santa Cruz) and mouse monoclonal antibody against bromodeoxyuridine (BrdU)(1:2; Becton Dickinson Immunocytometry Systems).

Secondary antibodies: fluorescein (FITC)-conjugated goat anti-mouse IgG (1:200; Biosource International), rhodamine (TRITC)-conjugated goat anti-mouse IgG (1:200; Molecular Probes) and fluorescein-conjugated affinity-purified goat antibody against mouse IgM (1:200; ICN Pharmaceuticals), FITC-conjugated goat anit-rabbit IgG (1:200; MBL, Japan) and AMCA-conjugated goat anti-rabbit IgG (1:200; Chemicon).

### (4) Immunocytochemistry

The cells were fixed in 4% paraformaldehyde in PBS containing 4% sucrose for 30 min. For c-Met immunofluorescence staining, the cells were fixed in 75% cold methanol, washed in PBS, incubated in blocking solution (2% skim milk, 1% normal goat serum, 0.2% BSA and 0.2% Triton X-100 in PBS) for 2 hr. For triple-labeling immunostaining, primary antibodies (anti-MAP-2 and anti-GFAP) were diluted in PBS containing 2% skim milk and 0.2% Triton X-100. The cells on the coverslips were incubated for 2 hr at 37°C, and the secondary antibodies were added and incubated for an additional 2 hr at 37°C. The cells were subsequently incubated with mouse IgM monoclonal antibody against O₄ for 1hr at 37 °C, and then were incubated with fluorescein-conjugated affinity-purified goat antibody to mouse IgM for another 1 hr at 37°C. Finally, the coverslips were washed twice with PBS, and then Hoechst (10 mM) was added and incubated for 5 min at room temperature, and washed twice in PBS. A rapid water wash preceded the mounting on glass slides with Vectoshield (Vector Laboratories).

### (5) BrdU labeling and detection

The incorporation of BrdU was determined by adding 10 µM BrdU (Sigma-Aldrich) to cultures of secondary neurospheres grown for 5 days in the presence of different growth factors. Twelve hours after BrdU addition, the cells were collected, washed with culture medium, mechanically dissociated, re-suspended in differentiation medium (growth medium plus 1% FBS) and plated onto poly-D-lysine coated coverslips in 24-well plates (Falcon). The cells were fixed 12 hr later in cold 75% methanol for 20 min, denatured in 2M HCl for 30 min, and then washed twice with PBS. Next, the cells were incubated with anti-BrdU for 30 min at 37 °C, and then washed with PBS two times. The cells were incubated with FITC-conjugated goat anti-mouse IgG for 30 min at 37°C. Finally, the cells were washed twice with PBS, and 0.04 mg/ml propidium iodide (Molecular Probes) was added. The cells were then incubated for 5 min at room temperature, and then washed twice with PBS.

### (6) TUNEL assay

To evaluate the number of apoptotic cells in the growing condition, secondary neurospheres were fixed in 1% paraformaldehyde. On the other hand, to evaluate the number of apoptotic cells in the differentiating condition, the cells were plated on poly-D-lysine coated coverslips in 24-well plates (Falcon) in differentiation medium for 6 days, and then fixed in 1% paraformaldehyde. The cells were stained using TUNEL kit (ApopTag Fluorescein kits, INTERGEN).

### (7) Cell counts and statistical analysis

Fluorescence was detected and photographed under a fluorescence microscope with a high resolution digital camera (DMRA, Q-Fish system, Leica, Germany). The immunoreactivity and number of cells in ten visual fields (50-100 cells per field) per coverslip were counted in a randomized fashion. Unpaired t-tests were used for assessing differences between experiments. All results are expressed as mean ±SEM.

### Results

### (1) Effect of HGF concentration on neurosphere formation and proliferation of NSCs derived from mouse embryonic brain

Neurospheres were not observed 7 days post-culture of primary E14 striatal cells at low density in the absence of growth factors (Fig. 1a and 1b). A significant number of neurospheres (63.8±44.8 cells/well) was observed at as low as 5 ng/ml of HGF. The number of neurospheres increased in a dose-dependent manner until 20 ng/ml of HGF and reached plateau at 50 ng/ml (Fig. 1a and 1b). The number of neurospheres formed by HGF was less than that obtained by the addition of FGF-2, EGF, or their combination at any concentration (Fig. 1b). The addition of 20 ng/ml of HGF to FGF-2, EGF, or their combination, significantly increased the number of neurospheres (without HGF: FGF-2 (341.3±89.6 cells/well), EGF (146.3±28.7 cells/well), FGF-2+EGF (507±95.7 cells/well); with HGF: FGF-2 (745.9±115.1 cells/well), EGF (511.9±43.5), FGF-2+EGF (1218.8±143.6 cells/well))(Fig. 1a and 1b).

To determine the effects of HGF on the proliferation of NSCs, the size of neurospheres was measured using a phase-contrast microscopy. Neurospheres were not observed in the absence of HGF. In the presence of 20 ng/ml of HGF, neurospheres were observed, but their size was smaller than those generated with the addition of other growth factors. When HGF was added to the medium containing FGF-2, EGF, or their combination, the size of neurospheres increased significantly (Table 1). These results suggest that the proliferative effect of HGF on NSCs can be synergistic with the presence of other growth factors.

**Table 1.**

| Effects of HGF on the size and numbers of primary neurospheres | | | | | | | |
|---|---|---|---|---|---|---|---|
| | None | FGF-2 | | EGF | | FGF-2 + EGF | |
| HGF | + | - | + | - | + | - | + |
| 200µm ≦ | 50.1±8.1 | 128.0±12.1 | 413.3 ±42.3* | 90.9±8.2 | 435.4±82.2* | 221.7±44.2 | 809.2±55.3* |
| 100-200µm | 180.4±50.1 | 768.3±90.1 | 1395.0±111.2* | 590.9±54.2 | 1306.2±151.2* | 886.8±77.1 | 1402.5±101.3 * |
| ≦ 100 µm | 670.2±88.2 | 853.7±52.4 | 1291.7±85.4* | 818.2±99.5 | 1088.5±123.2 | * 1241.5±99.2 | 1618.3±98.3* |
| Total no, | 900.7±20.1 | 1750.0±55.1 | 3100.0±66.2* | 1500.0±65.1 | 2830.0±86.4* | 2350.0±54.2 | 3830.0±75.3* |

E14 striatal cells were plated 3x10⁵ cells per well in a 6-well plate in the presence of the indicated growth factors. The size and number of primary neurospheres were determined 7 days later from counts obtained in five different experiments. *: p<0.05 versus HGF(-).

### (2) Character of cells in neurosphere obtained by incubation with HGF

To determine the expression of the HGF receptor, c-Met, immunostaining was performed on both the cells in the neurosphere and those dissociated in the isolation with HGF. Most of the cells in the neurospheres and the dissociated cells from neurospheres were immunohistochemically confirmed to express c-Met Fig. 2a and 2b). The expression of c-Met protein on neurospheres isolated with HGF or FGF-2 and EGF, was also confirmed by Western blot analysis (data not shown). c-Met was also immunopositive in cells isolated with the other growth factors (data not shown). Cells from neurospheres isolated with 20 ng/ml of HGF were also immunopositive for nestin, a stem cell marker (Fig. 2c).

### (3) The effect of HGF on BrdU-incorporation of neural progenitor

To determine the mechanism underlying the proliferative effects of HGF on NSCs, neurospheres were co-incubated with 10 µM of BrdU for 12 hr and fixed. BrdU is a thymidine analog that incorporates into the DNA of dividing cells. The addition of HGF to the medium containing FGF-2, EGF, or their combination significantly increases the percentage of BrdU-positive cells (Fig. 3a). It is possible that HGF promotes the survival of NSCs through inhibiting cell death during the culture of NSCs. To explore the effect of HGF on the survival of NSCs, TUNEL staining was performed on neurospheres cultured in the medium with and without HGF. The addition of HGF decreased the number of TUNEL-positive cells in the neurosphere (Fig. 3b).

### (4) The effect of HGF on differentiation of neural progenitor

To elucidate the effect of HGF on the differentiation of NSCs, and also to verify that neurospheres formed with HGF are truly NSCs, the ability of NSCs to differentiate into neurons, astrocytes, oligodendrocytes and other cell-types was investigated. First, the secondary neurospheres that were cultured in the medium containing 20 ng/ml of HGF for 7 days were dissociated, plated on coverslips with 1% FBS with and without 20 ng/ml of HGF, and then were incubated for 7 days. The cells were immunostained with neuronal marker, MAP2 (red), glial marker, GFAP (green) and nuclear marker, Hoechst (blue) (Fig. 4). The number of immunopositive cells for each marker was counted and their percentage in the whole cells was calculated. Interestingly, when HGF was added to the medium during differentiation, neurons were obtained more than astrocytes (without HGF: neuron (35.8±11.7%), astrocyte (43.1±16.0%); with HGF: neuron (52.5±7.9%), astrocyte (35.2±8.9%)) (Fig. 5a). Similar percentage were obtained when the cells were isolated with FGF-2 and EGF (without HGF: neuron (28.5±12.7%), astrocyte (50.8±11.9%); with HGF: neuron (53.5±8.9%), astrocyte (32 .6±7.9%) ) (Fig. 5b). When 20 ng/ml of HGF was added to the primary culture medium of cells from the brain, there was a tendency that more neurons existed after differentiation in the medium containing 1% FBS without HGF (Fig. 5c). However, when HGF was added to the medium containing 1% FBS for differentiation, a similar percentage of neurons was obtained (Fig. 5c right bars). These results suggest that HGF promotes differentiation into neurons upon addition to the medium of differentiation.

### Industrial Applicability

A medium containing hepatocyte growth factor (HGF) was shown to be capable of inducing neurosphere formation. Furthermore, the addition of HGF to a culture medium containing FGF-2, EGF, or both was shown to increase both the size and number of newly formed neurospheres. Thus, the present invention describes growth medium comprising HGF for culturing neural stem cells (NSCs) and provides methods for culturing the cells using the culture medium. Culturing an NSC or a cell population containing an NSC in an HGF-comprising growth medium provides a cell population enriched in NSCs or a cell population of differentiated NSCs. Such cell populations can be used to treat neurological disorders, such as epilepsy, head trauma, stroke, amyotrophic lateral sclerosis, Parkinson's disease, Alzheimer's disease and Huntington's disease.

HGF was found to promote proliferation and neuronal differentiation of NSCs isolated from E14 mouse embryos. Further understanding the mechanisms underlying the effects of HGF on NSCs may lead to the development of new biological techniques or *in vivo* treatments to control and regulate the genesis and/or repair of the central nervous system (CNS) that, in turn, can be used as novel therapeutic approaches against injuries and disorders.

## Claims

1. A method for culturing neural stem cells comprising the step of culturing a neural stem cell or a population of cells comprising at least one neural stem cell in a growth medium comprising hepatocyte growth factor (HGF), with the proviso that the neural stem cell or a population of cells comprising at least one neural stem cell is not obtained from human embryo.

2. A method for proliferating neural stem cells comprising the step of culturing a neural stem cell or a population of cells comprising at least one neural stem cell in a growth medium comprising HGF under conditions that allow the proliferation of the neural stem cell, with the proviso that the neural stem cell or a population of cells comprising at least one neural stem cell is not obtained from human embryo.

3. A method for differentiating neural stem cells comprising the step of culturing a neural stem cell or a population of cells comprising at least one neural stem cell in a growth medium comprising HGF under conditions that allow the differentiation of the neural stem cell into a population of cells containing neurons and glia cells, with the proviso that the neural stem cell or a population of cells comprising at least one neural stem cell is not obtained from human embryo.

4. The method of any one of claims 1-3, wherein the growth medium further comprises (i) fibroblast growth factor-2 (FGF-2), (ii) epidermal growth factor (EGF), or (iii) FGF-2 and EGF, in addition to HGF.

5. The method of any one of claims 1-4, wherein the neural stem cell is derived from mammalian neural tissue selected from the group consisting of brainstem, cerebellum, cerebral cortex, midbrain, spinal cord and ventricular.

6. The method of any one of claims 1-5, wherein the neural stem cell is genetically modified.

## Patentansprüche

1. Ein Verfahren zum Kultivieren von neuralen Stammzellen umfassend den Schritt des Kultivierens einer neuralen Stammzelle oder einer Population von Zellen, die zumindest eine neurale Stammzelle umfassen, in einem Wachstumsmedium, das Hepatocyte Growth Factor (HGF) umfasst, unter der Maßgabe, dass die neurale Stammzelle oder eine Population von Stammzellen, welche zumindest eine neurale Stammzelle umfasst, nicht von einem menschlichen Embryo erhalten wurde.

2. Ein Verfahren zum Proliferieren von neuralen Stammzellen, umfassend den Schritt des Kultivierens einer neuralen Stammzelle oder einer Population von Zellen, die zumindest eine neurale Stammzelle umfasst, in einem Wachstumsmedium, das HGF umfasst, unter Bedingungen, welche die Proliferation der neuralen Stammzelle ermöglichen, unter der Maßgabe, dass die neurale Stammzelle oder eine Population von Zellen, die zumindest eine neurale Stammzelle umfasst, nicht von einem menschlichen Embryo erhalten wurde.

3. Ein Verfahren zum Differenzieren von neuralen Stammzellen, umfassend den Schritt des Kultivierens einer neuralen Stammzelle oder einer Population von Zellen, die zumindest eine neurale Stammzelle umfasst, in einem Wachstumsmedium, das HGF umfasst, unter Bedingungen, welche die Differentiation der neuralen Stammzelle in eine Population von Zellen ermöglicht, welche Neuronen oder Gliazellen einschließt, unter der Maßgabe, dass die neurale Stammzelle oder eine Population von Zellen, welche zumindest eine neurale Stammzelle umfasst, nicht von einem menschlichen Embryo erhalten wurde.

4. Das Verfahren gemäß irgendeinem der Ansprüche 1 bis 3, wobei das Wachstumsmedium des weiteren folgendes umfasst:
(i) Fibroblast Growth Factor-2 (FGF-2),
(ii) Epidermal Growth Factor (EGF), oder
(iii) FGF-2 und EGF,
zusätzlich zu HGF.

5. Das Verfahren gemäß irgendeinem der Ansprüche 1 bis 4, wobei die neurale Stammzelle abgeleitet ist von einem neuralen Säugetiergewebe, ausgewählt aus der Gruppe bestehend aus Hirnstamm-, Cerebellum-, cerebralem Cortex-, Mittelhirn-, Rückenmarks- und ventrikulärem Gewebe.

6. Das Verfahren gemäß irgendeinem der Ansprüche 1 bis 5, wobei die neurale Stammzelle genetisch modifiziert ist.

## Revendications

1. Procédé de culture de cellules souches neurales comprenant l'étape consistant à cultiver une cellule souche neurale ou une population de cellules comprenant au moins une cellule souche neurale dans un milieu de croissance comprenant un facteur de croissance des hépatocytes (HGF), à la condition que la cellule souche neurale ou une population de cellules comprenant au moins une cellule souche neurale ne soit pas obtenue à partir d'un embryon humain.

2. Procédé de prolifération de cellules souches neurales comprenant l'étape consistant à cultiver une cellule souche neurale ou une population de cellules comprenant au moins une cellule souche neurale dans un milieu de croissance comprenant un HGF dans des conditions qui permettent la prolifération de la cellule souche neurale, à la condition que la cellule souche neurale ou une population de cellules comprenant au moins une cellule souche neurale ne soit pas obtenue à partir d'un embryon humain.

3. Procédé de différenciation de cellules souches neurales comprenant l'étape consistant à cultiver une cellule souche neurale ou une population de cellules comprenant au moins une cellule souche neurale dans un milieu de croissance comprenant un HGF dans des conditions qui permettent la différenciation de la cellule souche neurale dans une population de cellules contenant des neurones et des cellules gliales, à la condition que la cellule souche neurale ou une population de cellules comprenant au moins une cellule souche neurale ne soit pas obtenue à partir d'un embryon humain.

4. Procédé selon l'une quelconque des revendications 1 à 3, dans lequel le milieu de croissance comprend en outre (i) le facteur de croissance des fibroblastes 2 (FGF-2), (ii) un facteur de croissance de l'épiderme (EGF), ou (iii) le FGF-2 et un EGF, en plus de l'HGF.

5. Procédé selon l'une quelconque des revendications 1 à 4, dans lequel la cellule souche neurale est dérivée de tissus neuraux de mammifère choisis dans le groupe constitué par le tronc cérébral, le cervelet, le cortex cérébral, le mésencéphale, la moelle épinière et le ventricule.

6. Procédé selon l'une quelconque des revendications 1 à 5, dans lequel la cellule souche neurale est génétiquement modifiée.
